# EUROPÄISCHE PATENTSCHRIFT

(11) **EP 2 951 573 B1**
(45) Veröffentlichungstag und Bekanntmachung des Hinweises auf die Patenterteilung: **29.11.2017**
(21) Anmeldenummer: 14700928.6
(22) Anmeldetag: 20.01.2014
(51) Int. Cl.: G01N 33/00

(54) **RESTÖL-MESSGERÄT**
DEVICE FOR MEASURING RESIDUAL OIL
APPAREIL DE MESURE DE RESTES D'HUILE

(30) Priorität: 30.01.2013 DE 102013100950
(43) Veröffentlichungstag der Anmeldung: 09.12.2015
(73) Patentinhaber: Friedrich, Martin, 79843 Löfflingen (DE)
(72) Erfinder: Friedrich, Martin, 79843 Löfflingen (DE)
(74) Vertreter: Patentanwälte Bauer Vorberg Kayser
(86) Internationale Anmeldenummer: PCT/EP2014/051041
(87) Internationale Veröffentlichungsnummer: WO 2014/118028

(56) Entgegenhaltungen:
- WO-A2-2009/152811
- DE-A1-102010 020 110
- US-A- 4 102 648
- US-A1- 2012 279 277
- JAVIER G MONROY ET AL: "Calibration of MOX gas sensors in open sampling systems based on Gaussian Processes", SENSORS, 2012 IEEE, IEEE, 28. Oktober 2012 (2012-10-28), Seiten 1-4, XP032308910, DOI: 10.1109/ICSENS.2012.6411464 ISBN: 978-1-4577-1766-6

## Beschreibung

Die vorliegende Erfindung betrifft ein Messgerät und ein Verfahren zur Erfassung des Kohlenwasserstoffanteils in Gasen.

Derartige Messgeräte sind mit verschiedenen Sensortechnologien bekannt und dienen der Erfassung des Gehaltes von Öl, Kohlenwasserstoffen und oxidierbaren Gasen beispielsweise in Luft oder Druckluft.

US2012/279277 offenbart ein Messgerät zur Erfassung von Kohlenwasserstoffanteilen in einem Katalysatorgasstrom, wobei der Sensor als Photoionisationssensor ausgeführt ist und diskontinuierlich Messungen durchführt.

Häufig werden beispielsweise elektrisch beheizbare Metalloxid-Halbleitergassensoren mit Halbleiter-Oxidmaterialien verwendet, die im beheizten Zustand ihren elektrischen Widerstand in Abhängigkeit von der Menge der in der Luft enthaltenen Kohlenwasserstoffe verändern. Die wichtigsten Vorteile von Metalloxyd-Halbleitergassensoren sind die sehr hohe Empfindlichkeit und damit die Möglichkeit auch geringste Kohlenwasserstoffanteile bis in den ppt-Bereich messen zu können. Sie weisen eine sehr lange Standzeit, eine sehr gute Langzeitstabilität auf und die Anschaffungskosten sind eher gering.

Nachteilig bei Metalloxyd-Halbleitergassensoren ist allerdings, dass sie eine exponentielle Kennlinie aufweisen, wodurch sich ihr Offset-Punkt schwer bestimmen lässt. Die Messergebnisse sind relativ schlecht reproduzierbar und die Sensoren weisen hohe Querempfindlichkeiten gegen Wasserdampf und anorganische Gase auf. Die Einstellzeiten bis zum endgültigen Wert sind hoch und die Erholungszeiten bei einer Kalibrierung mit Nullluft bis zum Erreichen der Nulllinie sind relativ lang.

Eine weitere Methode ist die Erfassung der Kohlenwasserstoffkonzentration mittels Photoionisation. Dabei werden die Kohlenwasserstoffe mit ultraviolettem Licht bestrahlt. Die Energiemenge des Lichts muss dabei so hoch sein, dass Elektronen aus dem Kohlenwasserstoffmolekül herausgetrieben werden. Deren Anzahl lässt sich elektronisch messen. Photoionisationssensoren weisen eine gute Langzeitstabilität, eine relativ geringe Querempfindlichkeit gegen Wasserdampf und anorganische Gase auf. Die Einstellzeiten bis zum endgültigen Wert sind kurz, ebenso die Erholzeiten bei einer Kalibrierung mit Nullluft, bis die Nulllinie erreicht wird. Die Kennlinie ist linear, wodurch eine hohe Reproduzierbarkeit gegeben ist.
Nachteilig bei derartigen Sensoren ist jedoch deren geringe Empfindlichkeit, was insbesondere im gering konzentrierten Bereich relevant ist. Ohne Prüfgas lässt sich auch der Alterungszustand nicht sicher bestimmen, die Standzeit ist aber ohnehin mit etwa einem Jahr eher gering. Durch Verschleiß nimmt die Messgenauigkeit ab, weswegen auch die Wartungskosten hoch sind. Weiterhin sind auch die Anschaffungskosten von Photoionisationssensoren relativ hoch.

Die mittels Photoionisationssensoren generierten Messwerte lassen nur indirekt auf die gemessene Stoffmenge schließen, da die Messwerte auch vom molekularen Aufbau der Verbindung abhängig sind und selbst bei gleichen Summenformeln recht stark variieren. Sofern die zu messende Verbindung aber konstant, bekannt und möglichst auch einheitlich ist, lässt sich die Konzentration des Kohlenwasserstoffanteils relativ zuverlässig messen. Allerdings sinkt die Messgenauigkeit mit abnehmender Konzentration an Kohlenwasserstoffen. Insbesondere steigt dabei der Einfluss des Feuchtegehalts der Luft. Mit abnehmendem Kohlenwasserstoffanteil wird der Einfluss der Luftfeuchte zunehmend größer, Messungen von Kohlenwasserstoffanteilen im unteren mg/m³-Bereich und insbesondere im µg/-m³-Bereich sind nicht ausreichend genau durchzuführen.

Für die unterschiedlichen Anwendungen von Druckluft werden unterschiedliche Grenzwerte für den Ölanteil gefordert. Ölanteile bestehen aus tröpfchenförmigen Ölerosolen und aus Öldämpfen. Ölerosole und Öldämpfe können durch verschiedene Verfahren aus dem Druckluftstrom teilweise oder weitgehend eliminiert werden.

Die Aufgabe der Erfindung besteht darin, ein Messgerät zur Erfassung des Gehalts von Öl, Kohlenwasserstoffen und oxidierbaren Gasen in Gasen zu schaffen, dass auch geringste dauerhaft Konzentrationen zuverlässig misst. Mögliche Messfehler sollen leicht zu ermitteln und korrigierbar sein. Weiterhin ist es Aufgabe der Erfindung ein gegenüber dem Stand der Technik verbessertes Verfahren zur Erfassung des Gehaltes von Öl, Kohlenwasserstoffanteilen und oxidierbaren Gasen in Gasen bereit zu stellen.

Die Aufgabe wird gelöst, durch Messgerät zur Erfassung von Kohlenwasserstoffanteilen in Gasen, aufweisend
- einen ersten Sensor zur Bestimmung des Anteils an Kohlenwasserstoff in einem Messgasstrom und zur Erzeugung eines entsprechenden ersten Messergebnisses,
- einen zweiten Sensor zur Bestimmung des Anteils an Kohlenwasserstoff im Messgasstrom und zur Erzeugung eines entsprechenden zweiten Messergebnisses,
- eine Auswerteinheit zur Auswertung der Messergebnisse der beiden Sensoren
- eine Katalysatoreinheit (34) zur Erzeugung eines Katalysatorgasstroms (36)
   wobei
- der erste Sensor als Metalloxid-Halbleitergassensor ausgeführt ist und kontinuierlich Messungen durchführt,
- der zweite Sensor als Photoionisationssensor ausgeführt ist und diskontinuierlich Messungen durchführt.
- der Katalysatorgasstrom (36) dem zweiten Sensor (24) zuführbar ist.

Weiterhin wird die Aufgabe durch ein Verfahren zum Erfassen des Kohlenwasserstoffanteils in einem Gasstrom gelöst, das gekennzeichnet ist durch die Verfahrensschritte:
- kontinuierliches Zuleiten eines Messgasstroms an einen ersten Sensor, der als Metalloxid-Halbleitergassensor ausgeführt ist,
- Bestimmen des Anteils an Kohlenwasserstoff im Messgasstrom und Erzeugen eines ersten Messergebnisses durch den ersten Sensor,
- diskontinuierliches Zuleiten des Messgasstrom an einen zweiten Sensor (24), der als Photoionisationssensor ausgeführt ist,
- Bestimmen des Anteils an Kohlenwasserstoff im Messgasstrom und Erzeugen eines zweiten Messergebnisses durch den zweiten Sensor,
- Auswerten der Messergebnisse der beiden Sensoren,
- Erzeugen eines Katalysatorgasstroms (36), wobei dem zweiten Sensor (24) dann der Katalysatorgasstrom (36) zugeführt wird, wenn der zweite Sensor (24) abgeschaltet oder unbenutzt ist.

Das erfindungsgemäße Messgerät kombiniert erstmals die beiden unterschiedlichen Sensoren, die sich üblicherweise in einem Messgerät eher ausschließen. Es wurde bisher überflüssig angesehen, beide Sensoren in nur einem Messgerät zu verwenden.

Der Metalloxid-Halbleitergassensor, kurz MOX-Sensor, bietet als Vorteil gegenüber dem Photoionisationssensor, kurz PID-Sensor, die lange Lebenserwartung ohne Wartung bei geringen Anschaffungskosten. Für ein Öldampf-Messgerät lassen sich theoretisch mehrere Jahre Einsatz ohne Wartung und Rekalibrierung realisieren.

Der PID-Sensor ist bezüglich Querempfindlichkeit, Reproduzierbarkeit und Langzeitstabilität der genauere Sensor, er ermöglicht präzisere Messungen und weist eine geringere Querempfindlichkeit auf.

Ein wesentlicher Aspekt der Erfindung besteht darin, dass die beiden Sensoren unterschiedlich genutzt werden. Der MOX-Sensor ist dauerhaft in Betrieb und führt kontinuierlich Messungen durch. Der PID-Sensor dagegen misst nur in zyklischen Intervallen, zum Beispiel einmal täglich, zusätzlich und parallel zum MOX-Sensor. Die ermittelten Messwerte des PID-Sensors dienen dann dazu, beispielsweise eine Steigung oder den Offset des MOX-Sensors zu korrigieren. Die Messung des PID-Sensors erfolgt dabei unmittelbar nach einer zuvor vorzugsweise automatisch durchgeführten Nullluft-Kalibrierung.

Durch die diskontinuierliche Zuschaltung des PID-Sensors wird eine wesentlich längere Standzeit dieses ansonsten eher kurzlebigen Sensors erreicht.

Beide Sensoren weisen eine relativ hohe Querempfindlichkeit gegen Wasser auf. Bei MOX-Sensoren führt diese Querempfindlichkeit zu einer Verschiebung des Arbeitspunkts auf dessen exponentiellen Kennlinie und damit zu einem Offset- und/oder Gainfehler.

Beim PID-Sensor sind dagegen zwei Effekte bekannt, einmal der sogenannte Water-Quenching Effekt, der zu einem Steigungsfehler führt. Außerdem kann es zu Leckströmen aufgrund von Anlagerungen oder Verschmutzungen am Elektroden-Stack kommen, die zu einem feuchteabhängigen Offset-Fehler führen. Erfindungsgemäß kann deshalb am Gaseintritt des Messgeräts ein Trocknungselement, beispielsweise eine Membrane vorgesehen, welche den Wassergehalt signifikant reduziert. Hierfür können bekannte Membrantrockner verwendet werden, die die Luft selbst trocknen und expandierte Spülluft für den Trocknungsprozess nutzen. Auch die Nutzung von Adsorptionstrocknern ist möglich.

Der Einsatz eines Trocknungselements im Ursprungsgasstrom (Messgasstrom), beispielsweise eines Membrantrockners weist sowohl Vorteile für die Nutzung des PID-Sensors als auch für die Nutzung des MOX-Sensors auf, was weiterhin vorteilhaft gegenüber dem Stand ist, der diese beiden Sensortypen üblicherweise nicht kombiniert.

Grundsätzlich ist auch die Verwendung eines Adsorbtionstrockners möglich. Wesentlich ist, dass das Trocknungselement eine hohe Trocknungsleistung aufweist, die durch Adsorbtionstrockner oftmals nicht ausreichend gewährleistet ist.

Erfindungsgemäß ist darüber hinaus eine Katalysatoreinheit vorgesehen, die eine Offset-Stabilisierung des PID-Sensors mit Nullluft ermöglicht. Der PID-Sensor wird erfindungsgemäß immer dann mit einem Katalysatorgasstrom (katalysiertes Messgas) gespült, wenn er unbenutzt und abgeschaltet ist. Damit wird der PID-Sensors stets sauber gehalten und die Stabilität und Nutzungsdauer wird erhöht. Erst unmittelbar bevor der PID-Sensor parallel zum MOX-Sensor betrieben wird, wird der PID-Sensor unter Spannung gesetzt und dessen Lampe eingeschaltet. Nach ausreichender Stabilisierungszeit erfolgt ein automatischer Nullabgleich. Nach diesem Abgleich wird der Ursprungsgasstrom in einen ersten und zweiten Messgasstrom aufgeteilt, und dem PID-Sensor der zweite Messgasstrom zugeleitet. Der PID-Sensor vermisst diesen zweiten Messgasstrom dann parallel zum MOX-Sensor, der den ersten Messgasstrom vermisst.

Mit dem Messergebnis des PID-Sensors ist es möglich, den Offset des MOX-Sensors oder auch den Gain-Fehler des MOX-Sensors auszugleichen bzw. den MOX-Sensor zu kalibrieren.

Die Nutzung der Katalysatoreinheit erlaubt nicht nur eine reine Offset-Stabilisierung des PID-Sensors, sondern erfindungsgemäß auch die Kompensation der Querempfindlichkeit des MOX-Sensors und die Bestimmung des Offset-Punktes des MOX-Sensors auf seiner Kennlinie.

Die Kompensation der Querempfindlichkeit des MOX-Sensors mit Hilfe des PID-Sensors basiert auf den unterschiedlichen Arbeitsweisen der Sensoren. Der MOX-Sensor besitzt eine exponentielle Kennlinie, die bei der Herstellung des Sensors kalibriert wird und im Gerät hinterlegt ist. Der PID-Sensor dagegen arbeitet nahezu linear und wird mit Nullluft kalibriert. Der PID-Sensor kann somit den Nullwert bei einem Messvorgang um Klasse 1(ISO 8573: Öldampfanteile unter 0,01mg je m³ Gas) sehr genau messen.

Die Kompensation der Querempfindlichkeit des MOX wird durchgeführt, indem kalibrierte Punkte der Messwerte des PID-Sensors (auch die früher gemessenen Punkte) auf der exponentiellen Kurve des MOX-Sensors abgebildet werden, sofern die Messwerte schlechter als Klasse 1 sind. Der aktuelle Messwert des PID-Sensors wird dabei als Führungsgröße für eine statistische Wahrscheinlichkeitsberechnung aller zuvor bestimmten Kalibrierpunkte benutzt, die unwahrscheinlichen Punkte werden aus der Sammlung der Messwerte entfernt. Mit dem Mittelwert aller übrigen, also wahrscheinlichen Kalibrierpunkte wird die neue Steigung bestimmt und über ein Filter die Systemsteigung langsam nachgeführt.

Bei Messwerten besser als Klasse 1 wird hingegen der Offsetwert korrigiert, und der Arbeitspunkt auf der exponentiellen Kennlinie des MOX-Sensors für den Offsetwert bestimmt. Durch einen mathematischen Algorithmus ist es möglich, auch mit dem MOX-Sensor, der für Messung in Klasse 1 eigentlich eine viel zu hohe Querempfindlichkeit besitzt, bis hinunter zu Klasse 1 und besser zu messen.

Das Messgerät weist eine Auswerteinheit zur Auswertung der Messergebnisse auf. In einer ersten Ausführungsvariante können die beiden Sensoren mit einer einzigen Auswerteeinheit verbunden sein, denkbar ist aber auch, dass jedem Sensor eine eigene Auswerteeinheit zugeordnet ist. Die Auswerteeinheit weist einen Prozessor auf, der die notwendigen Berechnungen durchführt. Es können die einzelnen Gasströme oder die Summe aller Gasströme durch den Sensor bzw. die Sensoren gemessen oder von der Auswerteeinheit ausgewertet werden.

Weiterhin ist eine Anzeigeeinheit vorgesehen, die die Messergebnisse der Sensoren oder von der Auswerteeinheit berechnete Werte und/oder weitere Informationen anzeigt. Die Anzeigeeinheit kann vorteilhafterweise auch als Eingabeeinheit in Form eines Touchscreens ausgeführt sein.

Durch die Nutzung der beiden Sensoren wird auch erreicht, dass das Messgerät auch dann im Notbetrieb weiterbetrieben werden kann, wenn einer der beiden Sensoren ausfällt. Die Auswerteeinheit ist auch in der Lage, einen der beiden fehlerhaften Sensoren oder Gaswege selbstständig abzuschalten, so dass das Messgerät weiterhin einsatzbereit bleibt. Vorteilhafterweise gibt die Auswerteeinheit eine entsprechende Information über die Anzeigeeinheit aus, so dass das Messgerät vor Ausfall des zweiten Gasweges oder Sensors und dem damit verbundenen Gesamtausfall repariert werden kann. Ein wesentlicher Vorteil ergibt sich auch daraus, dass somit die entsprechende Reparatur zur Not während einer späteren ohnehin anstehenden Betriebspause durchführbar ist.

Letztendlich ist das Messgerät mit einem entsprechenden Fehleranalyseprogramm in der Lage, sämtliche Bauteile im Gasweg selbstständig zu überprüfen. Auch ein Spülen der Sensoren mit Referenzgas kann in regelmäßigen Zyklen oder aufgrund eines abweichenden oder auffälligen Messergebnisses vom Messgerät selbstständig eingeleitet werden.

Das erfindungsgemäße Messgerät kann weiterhin derart ausgeführt sein, dass beide Sensoren jeweils mit einem Referenzgasstrom, beispielsweise aus einer Vorratsflasche, beaufschlagt werden können. Neben dem unveränderten Messgas und dem katalysierten Messgas wird also weiterhin regelmäßig ein Referenzgasstrom, beispielsweise zur Neufestlegung der vom Sensor ausgehenden Signalstärke verwendet. Das Kalibriergas (z.B. Isobuten) weist einen definierten Kohlenwasserstoffanteil auf, allerdings keine oder nur eine ausgesprochen geringe Feuchtigkeit. Erfindungsgemäß ist es somit möglich, die Veränderung der Signalstärke und Messempfindlichkeit durch Alterung und Verschmutzung des Messgeräts zuverlässig auszugleichen. Die Kalibriermessung kann in regelmäßigen Abständen automatisch erfolgen, sie kann aber auch jederzeit vom Anwender eingeleitet werden. Insbesondere kann sie im Rahmen der Fehleranalyse vom Fehleranalyseprogramm genutzt werden. Die im Rahmen der Kalibriermessung ermittelten Daten werden gespeichert und können jederzeit abgerufen und von der Auswerteinheit genutzt werden.

Erfindungsgemäß können der PID-Sensor und der MOX-Sensor zur Gain-Kalibrierung gleichzeitig mit Referenzgas beaufschlagt werden. Diese Funktionalität kann zum einen für eine zyklische Autokalibrierung des Geräts genutzt werden, als auch für eine Rekalibrierung im Rahmen eines Service. Das Referenzgas hat typischerweise eine Konzentration im oberen Messbereich, z.B. 500 ppb. Nach Beginn der Zuleitung des Referenzgases zu den beiden Sensoren wird der Gain-Wert beider Sensoren nach einer angemessenen Stabilisierungszeit kalibriert.

Bei der parallelen Zuleitung des Referenzgases an beide Sensoren kann während der Gain-Kalibrierung keine Messung von Kohlenwasserstoffanteilen erfolgen. Erfindungsgemäß kann aber für beide Sensoren ein separates Ventil vorgesehen sein, das die Zuleitung zu nur einem der beiden Sensoren zulässt, wodurch die die Gain Kalibrierung separat durchgeführt und der jeweils andere Sensor für die kontinuierliche Messung weiter benutzt werden kann.

Das Messgerät kann dann besonders kostengünstig hergestellt werden, wenn technische Einheiten in Form von Baublöcken zusammengefasst werden. Dies kann beispielsweise Ventile, Drosseln, Katalysatoren und Sensoren betreffen. Die entsprechenden Elemente und Bauteile bestehen beispielsweise aus Metall.

Der PID-Sensor weist eine Sensoreinheit mit Probenahmesonde auf, vorzugsweise nutzen beide Sensoren gemeinsam eine Probenahmesonde. Die Sensoreinheit ist dabei über ein Signalkabel oder kabellos mit der Auswerteeinheit verbunden. Die Probenahmesonde kann vorzugsweise von oben zentrisch in eine Steigleitung montiert sein, so dass sie mittig aus dem zu überwachenden Gasstrom Gas entnehmen kann. Die Sensoreinheit weist definierte Fließwiderstände auf, die für einen konstanten Druck und einen konstanten Volumenstrom der einzelnen Messgase sorgen und beispielsweise durch eine Drossel mit definierter Bohrung, bzw. aus einem Sintermetall gebildet sind. Diese sind besonders wartungsarm und einfach zu reinigen. Weiterhin ist eine Alarmfunktion vorgesehen, die dem Benutzer bei zu niedrigem oder zu hohem Druck der Gasströme visuell oder akustisch informiert.

Die Durchflussmenge der verschiedenen Gasströme kann mit entsprechenden Drosseln, Ventilen oder Durchflussreduktoren beeinflusst werden. Diese sind vorzugsweise austauschbar und in einer besonders vorteilhaften Ausführungsvariante regelbar, um damit zum Einen die Durchflussmenge zu den Sensoren einstellen und zum Anderen die gewünschten Mischverhältnisse der zu mischenden Gasströme zuverlässig gewährleisten zu können.

Dadurch, dass die Ventile im Gasweg einzeln schaltbar sind, ist auch möglich, Referenzluft und Messluft gleichzeitig auf den Sensor zu schalten und das Messgas zu verdünnen. Damit kann der Messbereich nach oben erweitert werden, wenn die Messluft extrem stark verschmutzt ist.

Als Katalysatorgaseinheiten können übliche Oxidationskatalysatoren eingesetzt werden, denkbar sind aber auch andere Vorrichtungen oder Verfahren zur Bereitstellung von Gasen mit den gewünschten Eigenschaften. Als Oxidationskatalysator dient beispielsweise platinierte Quarzwolle, die problemlos in einen dafür vorgesehenen Behälter einführbar ist. Denkbar ist auch die Nutzung von Aktivkohle. In einer besonders vorteilhaften Variante ist der Referenzgaserzeuger in das Messgerät integriert, wodurch vor Ort lediglich die verschiedenen Fluid bzw. Gaszuführungen angeschlossen werden müssen.

Das Messgerät weist somit sämtliche Anschlüsse für entsprechende Gasleitungen und auch den elektrischen Anschluss auf, sodass es vor Ort flexibel an beliebigen Orten installierbar ist. Insbesondere die erfindungsgemäße Aufteilung des Messgeräts in die Sensoreinheit mit Sensoren, beispielsweise der Probenahmesonde im Falle des Prinzips der Photoionisation und die Auswerteeinheit mit Bedienoberfläche (Display) erweitert die Möglichkeiten einer räumlich flexiblen Aufstellung vor Ort zusätzlich. Die Auswerteeinheit mit Bedienoberfläche baut klein und kann nahezu überall, vorteilhafterweise an einer gut zugänglichen Position installiert werden, während die etwas größere Sensoreinheit räumlich getrennt von der Auswerteeinheit an der Messgasentnahmestelle angeordnet sein kann. Aber auch die Kombination beider Komponenten ist denkbar und bringt Vorteile. Das Gerät ist dann kompakt und preisgünstig und da der Öldampf in der Gasphase vorliegt kann auch ein Schlauch oder Rohr zur Zuleitung verwendet werden. Außerdem ist die kombinierte Einheit für Wartung einfacher zugänglich.

Das erfindungsgemäße Messgerät kann vorzugsweise mit einem ölfrei verdichtenden Kompressor zur Herstellung von Druckluft oder Druckgas verwendet werden, denkbar ist aber auch die Verwendung mit einem ölgeschmierten Kompressor, wenn diesem ein entsprechender Katalysator nachgeschaltet ist. Für Wartungsarbeiten ist vorzugsweise ein Bypass vorgesehen. Grundsätzlich ist das Messgerät aber auch für weitere Verwendungsbereiche geeignet, beispielsweise für Druckgasflaschen.

Die Erfindung wird im Folgenden mit Bezug auf die beiliegenden Figuren näher erläutert. Dabei zeigen die Figuren lediglich eine vorteilhafte Ausführungsvariante in stark vereinfachter Prinzipdarstellung, die Erfindung soll keinesfalls auf diese beschränkt sein. Es zeigen:
- Fig. 1:: ein Gaswegschaltschema des Messgeräts,
- Fig. 2:: ein Messzyklusschema des Messgeräts,
- Fig. 3:: Kennlinie des MOX-Sensors zur Erläuterung der Kompensation der Querempfindlichkeit

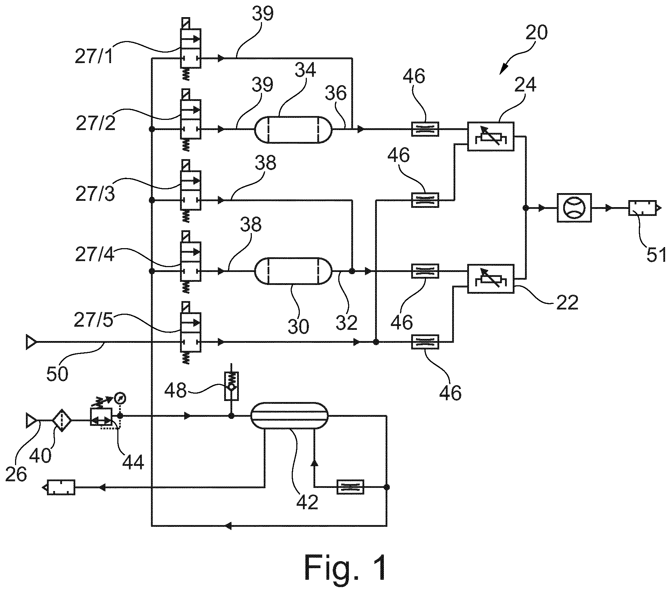

Figur 1 zeigt ein Gaswegschaltschema des Messgeräts 20. Dieses weist zwei Sensoren, als ersten Sensor 22 einen Metalloxid-Halbleitergassensor (im Folgenden MOX-Sensor) und als zweiten Sensor 24 einen Photoionisationsensor (im Folgenden PID-Sensor) auf.

Ein Ursprungsgasstrom 26 wird über Gasleitungen und mit Hilfe von Ventilen 27 in einen ersten Messgasstrom 38 und einen zweiten Messgasstrom 39 aufgeteilt.

Im gezeigten Ausführungsbeispiel ist dem zweiten Sensor 24 eine Katalysatoreinheit 34 vorgeschaltet, die einen Katalysatorgasstrom 36 erzeugt. Analog ist dem ersten Sensor 22 eine zweite Katalysatoreinheit 30 vorgeschaltet, die einen zweiten Katalysatorgasstrom 32 erzeugt.

Ein Filterelement 40 filtert und ein Trocknungselement 42 trocknet den Ursprungsgasstrom 26 und damit die beiden Messgasströme 38, 39. Das Trocknungselement 42 ist vorzugsweise als Membrantrockner ausgeführt.

Weiterhin sind ein Druckregler 44 und ein Sicherheitsventil 48 im Ursprungsgasstrom 26 vorgesehen. Drosseln 46, vorzugsweise Expansionsdrosseln sind den Sensoren 22, 24 vorgeschaltet.

Ein erstes Ventil 27-1 schaltet den zweiten Messgasstrom 39 und ein zweites Ventil 27-2 den Katalysatorgasstrom 36 zum zweiten Sensor 24. Ein drittes Ventil 27-3 schaltet den ersten Messgasstrom 38 und ein viertes Ventil 27-4 den zweiten Katalysatorgasstrom 32 zum ersten Sensor 22.

Über ein fünftes Ventil 27-5 kann den beiden Sensoren 22, 24 ein Referenzgasstrom 50 zugeleitet werden. Dieser stammt vorzugsweise aus einer extern angeschlossenen Gasflasche, wobei das Gas eine bekannte Konzentration an Kohlenwasserstoffen, beispielsweise im Bereich von 300 - 1000 ppb, vorzugsweise 500 ppb aufweist.

Das Messgerät weist optional einen Schalldämpfer 51 auf.

Der zweite Sensor 24 kann somit über das erste Ventil 27-1 und über das zweite Ventil 27-2 im Wechsel mit dem zweiten Messgasstrom 39 oder dem Katalysatorgasstrom 36 versorgt werden. Analog kann der erste Sensor 22 über das dritte Ventil 27-3 und das vierte Ventil 27-4 entsprechend mit dem ersten Messgasstrom 38 oder dem zweiten Katalysatorgasstrom 32 versorgt werden. Beiden Sensoren kann über das fünfte Ventil 27-5 ein Referenzgasstrom 50 zugeleitet bekommen.

Der Ursprungsgasstrom 26 wird über das Filterelement 40 von Partikeln gereinigt und über den Druckregler 44 beispielsweise auf 3,8 bar geregelt. Der Ursprungsgasstrom wird anschließend über das Trocknungselement 42 getrocknet, wobei der Anteil an Kohlenwasserstoff nicht verändert wird. Somit steht am Austritt des Trockenelements ein getrockneter Ursprungsgasstrom 26 mit einem Taupunkt von etwa minus 70° C und einem unveränderten Gehalt an Kohlenwasserstoffen bereit. Beide Sensoren 22, 24 können mit diesem getrockneten Ursprungsgasstrom 26 betrieben werden.

Während des Betriebs wird der erste Sensor 22, der MOX-Sensor, kontinuierlich mit dem ersten Messgasstrom 38 (also dem getrockneten Ursprungsgasstrom 26), beaufschlagt. Das erste Ventil 27-1 ist geschlossen, das zweite Ventil 27-2 geöffnet, so dass der zweite Sensor 24 dauerhaft mit dem Katalysatorgasstrom 36 gespült wird. Der zweite Sensor 24 ist dabei zunächst ausgeschaltet. Das dritte Ventil 27-3 ist geöffnet, das vierte Ventil 27-4 und das fünfte Ventil 27-5 sind geschlossen.

Zur Referenzmessung mit dem zweiten Sensor 24 wird dieser zunächst eingeschaltet. Nach ausreichender Stabilisierungszeit, d.h. konstanter Basislinie, wird dann ohne weiteres Schalten von Ventilen der Offset-Wert des zweiten Sensors 24 aufgenommen. Da das zweite Ventil 27-2 bereits geöffnet war, wurde der zweite Sensor 24 mit dem Katalysatorgasstrom 36, also Nullluft, gespült und ist kohlenwasserstofffrei.

Nach der Nullpunkt-Aufnahme schließt das zweite Ventil 27-2 und das erste Ventil 27-1 wird geöffnet. Der zweite Sensor 24, der PID-Sensor, wird nun mit dem zweiten Messgasstrom 39 betrieben und arbeitet parallel zum ersten Sensor 22.

Nachdem der zweite Sensor 24 Messwerte ermittelt hat, werden folgende Entscheidungen getroffen:
Liegt der Messwert unter einem bestimmten Wert (der typischerweise Klasse 1 entspricht, ca. 5 ppb), wird über einen Algorithmus der Offset-Punkt des ersten Sensors 22 korrigiert. Der Algorithmus berücksichtigt die exponentielle Kennlinie des ersten Sensors 22.

Liegt der Messwert über der Klasse 1, so wird über einen Algorithmus die Steigung der Kennlinie des ersten Sensors 22 korrigiert. Auch dieser Algorithmus berücksichtigt die exponentielle Kennlinie des ersten Sensors.

Nach dieser Referenzmessung des zweiten Sensors 24 wird dieser wieder auf Nullluft, also den Katalysatorgasstrom 36, geschaltet und die Betriebsspannung abgeschaltet.

Über das vierte Ventil 27-4 kann beim ersten Sensor 22 eine Nullpunkt-Kalibrierung durchgeführt werden. Dazu schließt sich das dritte Ventil 27-3 und öffnet sich das vierte Ventil 27-4. Der erste Sensor 22 wird somit mit dem zweiten Katalysatorgasstrom 32, also Nullluft, beaufschlagt. Nach ausreichender Stabilisierungszeit kann dessen Offset kalibriert werden.

Über das fünfte Ventil 27-5 können beide Sensoren 22, 24 gleichzeitig mit dem Referenzgasstrom 50 beaufschlagt werden. Dies kann zyklisch im Rahmen einer Autokalibrierung des Gerätes erfolgen, es ist aber auch eine Rekalibrierung im Rahmen einer Servicemaßnahme möglich.

Mit Hilfe des Referenzgasstrom 50 kann eine Gain-Kalibrierung durchgeführt werden. Zu diesem Zweck sind die ersten Ventile 27-1 bis 27-4 geschlossen und nur das fünfte Ventil 27-5 geöffnet. Nach angemessener Stabilisierungszeit kann der Gain-Wert beider Sensoren 22, 24 bestimmt werden.

Alternativ kann erfindungsgemäß ein weiteres Ventil vorgesehen sein, das eine getrennte Gain-Kalibrierung der beiden Sensoren 22, 24 ermöglicht.

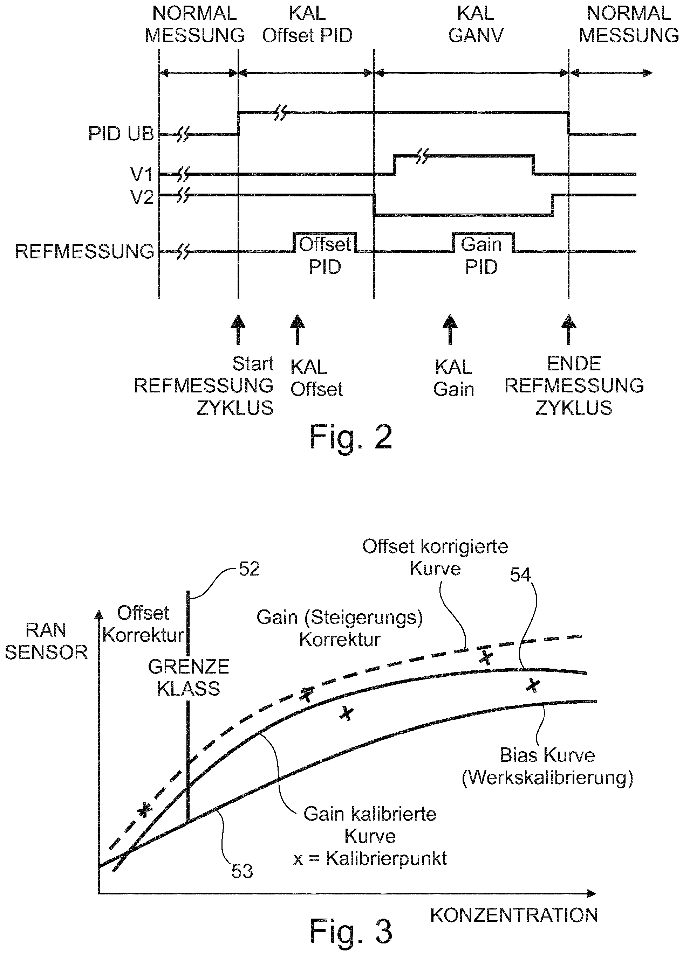

Figur 2 verdeutlicht den zeitlichen Verlauf des oben beschriebenen Messvorgangs.

Figur 3 verdeutlicht die Berechnung zur Kompensation der Querempfindlichkeit des ersten Sensors 22 auf Basis der Messergebnisse des zweiten Sensors 24.

Eine Klassengrenze 52 teilt die Kurve in einen Bereich zur Offset-Korrektur und einen Bereich zur Gain (Steigungs)-Korrektur auf. Neben der Nutzung der Klassengrenze 52 sind auch andere Algorithmen möglich, um Offset- und Gain oder sogar die exponentielle Kennlinie zu modifizieren.

Sind die vom zweiten Sensor 24 gemessenen Messwerte schlechter als Klasse 1, ist also die Konzentration höher, wird der aktuelle Messwert des zweiten Sensors als Führungsgröße für eine statistische Wahrscheinlichkeitsberechnung aller zuvor bestimmten Kalibrierpunkte oder Messwerte verwendet. Die unwahrscheinlichsten Messwerte werden dann aus der Sammlung der Messwerte entfernt und mit dem Mittelwert der übrigen Messwerte, die wahrscheinlicher sind, wird eine neue Steigung und damit eine gegenüber einer Basiskennlinie 53 korrigierte Kurve 54 bestimmt und über einen Filter die Systemsteigung langsam nachgeführt.

Sind die Messwerte besser als Klasse 1, wird eine Offset-Kalibrierung durchgeführt und der Arbeitspunkt auf der exponentielle Kennlinie des ersten Sensors 22 für den Offsetwert bestimmt. Somit ist es möglich mit dem ersten Sensor 22, der eigentlich eine viel zu hohe Querempfindlichkeit besitzt, bis hinunter zu Klasse 1 zu messen und zuverlässige Messergebnisse zu erhalten.

Die Erfindung ist nicht auf das beschriebene Ausführungsbeispiel beschränkt, dieses dient ausschließlich der Beschreibung und soll nicht einschränkend zu verstehen sein.

## Patentansprüche

1. Messgerät (20) zur Erfassung von Kohlenwasserstoffanteilen in Gasen, aufweisend
- einen ersten Sensor (22) zur Bestimmung des Anteils an Kohlenwasserstoff in einem ersten Messgasstrom (38) und zur Erzeugung eines entsprechenden ersten Messergebnisses,
- einen zweiten Sensor (24) zur Bestimmung des Anteils an Kohlenwasserstoff in einem zweiten Messgasstrom (39) und zur Erzeugung eines entsprechenden zweiten Messergebnisses,
- eine Auswerteinheit zur Auswertung der Messergebnisse der beiden Sensoren (22, 24),
- eine Katalysatoreinheit (34) zur Erzeugung eines Katalysatorgasstroms (36)
wobei
- der erste Sensor (22) als Metalloxid-Halbleitergassensor ausgeführt ist und kontinuierlich Messungen durchführt,
- der zweite Sensor (24) als Photoionisationssensor ausgeführt ist und diskontinuierlich Messungen durchführt,
- der Katalysatorgasstrom (36) dem zweiten Sensor (24) zuführbar ist.
P025n

2. Messgerät (20) nach Anspruch 1, **dadurch gekennzeichnet, dass** eine zweite Katalysatoreinheit (30) zur Erzeugung eines zweiten Katalysatorgasstroms (32) vorhanden ist, der dem ersten Sensor (22) zuführbar ist.

3. Messgerät (20) nach einem der Ansprüche 1 bis 2, **dadurch gekennzeichnet, dass** die Katalysatorgaseinheiten (30, 34) durch Oxidationskatalysatoren ausgebildet sind.

4. Messgerät (20) nach einem der Ansprüche 1 bis 3, **dadurch gekennzeichnet, dass** den beiden Sensoren (22, 24) ein Referenzgasstrom (50) mit einer Konzentration an Kohlenwasserstoffen im oberen Messbereich der beiden Sensoren (22, 24) zuführbar ist.

5. Messgerät (20) nach einem der Ansprüche 1 bis 4, **dadurch gekennzeichnet, dass** in Strömungsrichtung vor den Sensoren (22, 24) ein Filterelement (40) zur Filtration des Messgasstroms (26) vorhanden ist.

6. Messgerät (20) nach einem der Ansprüche 1 bis 5, **dadurch gekennzeichnet, dass** in Strömungsrichtung vor den Sensoren (22, 24) ein Trocknungselement (42) zur Trocknung der Messgasströme (38, 39) vorhanden ist.

7. Messgerät (20) nach einem der Anspruch 5, **dadurch gekennzeichnet, dass** das Trocknungselement (42) als Membrantrockner ausgeführt ist.

8. Verfahren zum Erfassen des Kohlenwasserstoffanteils in einem Gasstrom, **gekennzeichnet durch** die Verfahrensschritte,
- kontinuierliches Zuleiten eines ersten Messgasstroms (38) an einen ersten Sensor (22), der als Metalloxid-Halbleitergassensor ausgeführt ist,
- Bestimmen des Anteils an Kohlenwasserstoff im ersten Messgasstrom (38) und Erzeugen eines ersten Messergebnisses **durch** den ersten Sensor (22),
- diskontinuierliches Zuleiten eines zweiten Messgasstrom (39) an einen zweiten Sensor (24), der als Photoionisationssensor ausgeführt ist,
- Bestimmen des Anteils an Kohlenwasserstoff im zweiten Messgasstrom (39) und Erzeugen eines zweiten Messergebnisses **durch** den zweiten Sensor (24),
- Auswerten der Messergebnisse der beiden Sensoren (22, 24),
- Erzeugen eines Katalysatorgasstroms (36), wobei dem zweiten Sensor (24) dann der Katalysatorgasstrom (36) zugeführt wird, wenn der zweite Sensor (24) abgeschaltet oder unbenutzt ist.

9. Verfahren nach Anspruch 8, **dadurch gekennzeichnet, dass**
- der zweite Sensor (24) erst unmittelbar vor der Benutzung aktiviert wird,
- nach ausreichender Stabilisierungszeit ein automatischer Nullabgleich durchgeführt wird,
- nach dem Nullabgleich der Messgasstrom (39) zugeleitet wird.

10. Verfahren nach einem der Ansprüche 8 bis 9, **dadurch gekennzeichnet, dass** im Anschluss der parallelen Messung der beiden Sensoren (22, 24) die Messergebnisse der beiden Sensoren (22, 24) verglichen und der erste Sensor (22) bedarfsweise kalibriert wird.

11. Verfahren nach einem der Ansprüche 8 bis 10, **dadurch gekennzeichnet, dass** die Messgasströme (38, 39) vor dem Zuleiten an die beiden Sensoren (22, 24) mit Hilfe eines Filterelement (40) filtriert werden.

12. Verfahren nach einem der Ansprüche 8 bis 11 **dadurch gekennzeichnet, dass** die Messgasströme (38, 39) vor dem Zuleiten an die beiden Sensoren (22, 24) mit Hilfe eines Trocknungselement (42) getrocknet werden.

13. Verfahren nach einem der Ansprüche 8 bis 12, **gekennzeichnet durch** eine Kalibrierung der beiden Sensoren (22, 24) mit Hilfe eines Referenzgasstroms (50) mit einer Konzentration an Kohlenwasserstoffen im oberen Messbereich der beiden Sensoren (22, 24).

14. Verfahren nach einem der Ansprüche 8 bis 13, **gekennzeichnet durch** Kompensation einer Querempfindlichkeit des ersten Sensors (22), wenn die Messwerte des zweiten Sensors (24) schlechter als Klasse 1 (nach ISO 8573) sind, **durch**
a. Nutzung eines aktuellen Messergebnisses des zweiten Sensors (24) als Führungsgröße für eine statistische Wahrscheinlichkeitsberechnung früher bestimmter Kalibrierwerte des ersten Sensors (22),
b. Entfernen der unwahrscheinlichsten Kalibrierwerte aus der Sammlung der früheren Kalibrierwerte,
c. Bestimmung einer Steigung einer Messkurve aus einem Mittelwert, der sich aus der Sammlung der früheren Kalibrierwerte nach Entfernen der unwahrscheinlichsten Kalibrierwerte ergibt,
d. Nachführen einer Systemsteigung mit Hilfe eines Filters.

15. Verfahren nach einem der Ansprüche 8 bis 14, **gekennzeichnet durch** eine Korrektur eines Offsetwertes des ersten Sensors (22), wenn die Messwerte des zweiten Sensors (24) besser als Klasse 1 (nach ISO 8573) sind, **durch**
a. Bestimmen eines Arbeitspunktes auf einer Messkurve aus den Messergebnissen des ersten Sensors (22)
b. Berechnung von Werten zur Korrektur des Offsetwertes des ersten Sensors (22).
c. Berücksichtigung der exponentiellen Kennlinie des ersten Sensors (22) bei der Ermittlung des Offsetwertes.

## Claims

1. A measuring device (20) for detecting hydrocarbon contents in gases, comprising
- a first sensor (22) for determining the hydrocarbon content in a measuring gas flow (38) and for producing a corresponding first measurement result,
- a second sensor (24) for determining the hydrocarbon content in a second measuring gas flow (39) and for producing a corresponding second measurement result,
- an evaluation unit for evaluating the measurement results of the two sensors (22, 24),
- a catalyst unit (34) for producing a catalyst gas flow (36),
wherein
- the first sensor (22) is configured as a metal oxide semiconductor gas sensor and continuously carries out measurements,
- the second sensor (24) is configured as a photoionization sensor and discontinuously carries out measurements,
- the catalyst gas flow (36) can be fed to the second sensor (24).

2. The measuring device (20) according to claim 1, **characterized in that** a second catalyst unit (30) for the generation of a second catalyst gas flow (32), which can be fed to the first sensor (22), is provided.

3. The measuring device (20) according to any one of the claims 1 to 2, **characterized in that** the catalyst gas units (30, 34) are formed by oxidation catalysts.

4. The measuring device (20) according to any one of the claims 1 to 3, **characterized in that** a reference gas flow (50) with a hydrocarbon concentration in the upper measurement range of the two sensors (22, 24) can be fed to the two sensors (22, 24).

5. The measuring device (20) according to any one of the claims 1 to 4, **characterized in that** a filter member (40) for filtrating the measuring gas flow (26) is provided forward of the sensors (22, 24) in the flow direction.

6. The measuring device (20) according to any one of the claims 1 to 5, **characterized in that** a drying element (42) for drying the measuring gas flows (38, 39) is provided forward of the sensors (22, 24) in the flow direction.

7. The measuring device (20) according to any one of the claims 5, **characterized in that** the drying element (42) is configured as a membrane dryer.

8. A method for detecting the hydrocarbon content in a gas flow, **characterized by** the method steps:
- continuously feeding a first measuring gas flow (38) to a first sensor (22) configured as a metal oxide semiconductor gas sensor,
- determining the hydrocarbon content in the first measuring gas flow (38) and producing a first measurement result by means of the first sensor (22),
- discontinuously feeding a second measuring gas flow (39) to a second sensor (24) configured as a photoionization sensor,
- determining the hydrocarbon content in the second measuring gas flow (39) and producing a second measurement result by means of the second sensor (24),
- evaluating the measurement results of the two sensors (22, 24),
- producing a catalyst gas flow (36), wherein the catalyst gas flow (36) is fed to the second sensor (24) when the second sensor (24) is turned off or not used.

9. The method according to claim 8, **characterized in that**
- the second sensor (24) is activated only immediately before use,
- an automatic zero adjustment is carried out after a sufficient stabilization time,
- the measuring gas flow (39) is fed after the zero adjustment.

10. The method according to any one of the claims 8 to 9, **characterized in that**, subsequent to the parallel measurement of the two sensors (22, 24), the measurement results of the two sensors (22, 24) are compared and the first sensor (22) is calibrated if necessary.

11. The method according to any one of the claims 8 to 10, **characterized in that** the measuring gas flows (38, 39) are filtrated by means of a filter member (40) prior to being fed to the two sensors (22, 24).

12. The method according to any one of the claims 8 to 11, **characterized in that** the measuring gas flows (38, 39) are dried by means of a drying element (42) prior to being fed to the two sensors (22, 24).

13. The method according to any one of the claims 8 to 12, **characterized by** a calibration of the two sensors (22, 24) by means of a reference gas flow (50) with a hydrocarbon concentration in the upper measurement range of the two sensors (22, 24).

14. The method according to any one of the claims 8 to 13, **characterized by** a compensation of a cross sensitivity of the first sensor (22) if the measured values of the second sensor (24) are worse than Class 1 (according to ISO 8573), by
a. using a current measurement result of the second sensor (24) as a reference quantity for a statistical probability calculation of previously determined calibration values of the first sensor (22),
b. removal of the most improbable calibration values from the collection of the previous calibration values,
c. determination of a slope of a measurement curve from a mean value resulting from the collection of the previous calibration values after the removal of the most improbable calibration values,
d. adjustment of a system slope by means of a filter.

15. The method according to any one of the claims 8 to 14, **characterized by** a correction of an offset value of the first sensor (22) if the measured values of the second sensor (24) are better than Class 1 (according to ISO 8573), by
a. determining an operating point on a measurement curve from the measurement results of the first sensor (22),
b. calculation of values for correcting the offset value of the first sensor (22),
c. taking into account the exponential characteristic curve of the first sensor (22) in the determination of the offset value.

## Revendications

1. Instrument de mesure (20) pour capturer les taux d'hydrocarbures dans les gazes, comprenant
- un premier capteur (22) pour déterminer le taux d'hydrocarbures dans un premier courant de gaz de mesure (38) et pour produire un premier résultat de mesure approprié, un second capteur (24) pour déterminer le taux d'hydrocarbures dans un second courant de gaz de mesure (39) et pour produire un second résultat de mesure approprié,
- une unité d'analyse pour analyser les résultats de mesure des deux capteurs (22, 24), une unité de catalyseur (34) pour produire un courant de gaz catalyseur (36)
dans lequel
- le premier capteur (22) et configuré comme capteur de gaz à oxyde métallique semi-conducteur réalisant des mesures continues,
- le deuxième capteur (24) est configuré comme capteur photoionisation réalisant des mesures discontinues,
- le courant de gaz catalyseur (36) est conductible vers le second capteur (24).

2. Instrument de mesure (20) selon la revendication 1, **caractérisé par le fait, qu'**une seconde unité de catalyseur (30) pour produire un second courant de gaz catalyseur (32) est présente, qui est conductible vers le premier capteur (22).

3. Instrument de mesure (20) selon l'une des revendications 1 à 2, **caractérisé par le fait, que** les unités de catalyseur (30, 34) sont formées par des catalyseurs d'oxydation.

4. Instrument de mesure (20) selon l'une des revendications 1 à 3, **caractérisé par le fait, qu'**un courant de gaz de référence (SO) ayant une concentration des hydrocarbures dans la plage de mesure élevée de deux capteurs (22, 24) est conductible vers les deux capteurs (22. 24).

5. Instrument de mesure (20) selon l'une des revendications 1 à 4, **caractérisé par le fait, qu'**un élément de filtrage (40) pour le filtrage du courant de gaz de mesure (26) est présent dans la direction du flux en aval des capteurs (22, 24).

6. Instrument de mesure (20) selon l'une des revendications 1 à 5, **caractérisé par le fait, qu'**un élément de séchage (42) pour le séchage des courants de gaz de mesure (38, 39) est présent dans la direction du flux en aval des capteurs (22, 24).

7. Instrument de mesure (20) selon l'une de revendication 5, **caractérisé par le fait, que** l'élément de séchage (42) est ménagé en tant que sécheur à membrane.

8. Procédé pour capturer le taux d'hydrocarbures dans un courant de gaz, **caractérisé par** les étapes du processus consistant à
- conduire, de manière continue, un premier courant de gaz de mesures (38) vers un premier capteur (22), qui est configuré comme capteur de gaz à oxyde métallique semi-conducteur,
- déterminer le taux d'hydrocarbure dans le premier courant de gaz de mesure (38) et produire un premier résultat de mesure par le premier capteur (22),
- conduire, de manière discontinue, un second courant de gaz de mesure (39) vers un second capteur (24), qui est configuré comme capteur photoionisation,
- déterminer le taux d'hydrocarbure dans le second courant de gaz de mesure (39) et produire un second résultat de mesure par le second capteur (24),
- analyser le résultat de mesure des deux capteurs (22, 24),
- produire un courant de gaz de catalyseur (36), dans lequel le courant de gaz catalyseur (36) est conduit au second capteur (24) lorsque le deuxième capteur (24) est arrêté ou n'est pas utilisé.

9. Procédé selon la revendication 8, **caractérisé par le fait, que**
- le deuxième capteur (24) ne sera activé que immédiatement avant l'emploi,
- après un temps de stabilisation suffisant un réglage du zéro automatique est mise en oeuvre,
- après le réglage du zéro, le courant de gaz de mesure (39) y est conduit.

10. Procédé selon l'une des revendications 8 bis 9, **caractérisé par le fait, que**, à la suite de la mesure parallèle des deux capteurs (22, 24), les résultats de mesure des deux capteurs (22, 24) seront comparés et, le cas nécessaire, le premier capteur (22) sera calibré.

11. Procédé selon l'une des revendications 8 bis 10, **caractérisé par le fait, que** les courants de gaz de mesure (38, 39), avant les conduire vers les deux capteurs (22,24), sont filtrés à l'aide d'un élément de filtrage (40).

12. Procédé selon l'une des revendications 8 bis 11 **caractérisé par le fait, que** les courants de gaz de mesure (38, 39), avant les conduire vers les deux capteurs (22,24), sont séchés à l'aide d'un élément de séchage (42).

13. Procédé selon l'une des revendications 8 bis 12, **caractérisé par** un calibrage des deux capteurs (22, 24) à l'aide d'un courant de gaz de référence (50) ayant une concentration d'hydrocarbures dans la plage de mesure élevée des deux capteurs (22,24).

14. Procédé selon l'une des revendications 8 bis 13, **caractérisé par** la compensation d'une sensibilité croisée du premier capteur (22), lorsque les valeurs mesurées du second capteur (24) sont plus mauvaises que classe 1 (selon ISO 8573), par le fait de
a. utiliser un résultat de mesure actuel du second capteur (24) comme grandeur de contrôle pour un calcul des probabilités statistique des valeurs de calibrage du premier capteur (22) déterminées auparavant,
b. éliminer les valeurs de calibrage les plus improbables de la collection des valeurs de calibrage antérieures,
c. déterminer une pente d'une courbe de mesure à partir d'une valeur moyenne résultant de la collection des valeurs antérieures après l'élimination des valeurs de calibrage les plus improbables,
d. tracer une pente de système à l'aide d'un filtre.

15. Procédé selon l'une des revendications 8 bis 14, **caractérisé par** une correction d'une valeur de décalage du premier capteur (22), lorsque les valeurs de mesure du second capteur (24) sont meilleures que classe 1 (selon ISO 8573), par le fait de
a. déterminer un point de travail sur une courbe de mesure à partir des résultats de mesure du premier capteur (22)
b. calculer de valeurs pour la correction de la valeur de décalage du premier capteur (22).
c. tenir compte de la courbe caractéristique exponentielle du premier capteur (22) lors de la détermination de la valeur de décalage.
